# EUROPEAN PATENT APPLICATION

(11) **EP 4 617 638 A1**
(43) Date of publication of application: **17.09.2025**
(21) Application number: 25162132.2
(22) Date of filing: 06.03.2025
(51) Int. Cl.: G01M 99/00, A61M 5/20, A61M 5/32

(54) **INJECTION NEEDLE CAP REMOVAL TOOLS AND INJECTION TESTING SYSTEMS**

(30) Priority: 12.03.2024 US 202463564223 P; 04.03.2025 US 202519069581
(71) Applicant: Illinois Tool Works Inc., Glenview IL 60025 (US)
(72) Inventor: DIEP, Sonny Tran, Glenview, 60025 (US)
(74) Representative: HGF

(57) **Abstract**

Disclosed example cap removal tools include: a cap removal adapter having a cap removal aperture; and a base, comprising: first alignment features configured to align the cap removal tool with an actuator of the autoinjector testing system; and second alignment features configured to removably attach the cap removal adapter to the base and align the cap removal adapter with the actuator.

## Description

### RELATED APPLICATIONS

The present application claims the benefit of U.S. Provisional Patent Application Serial No. 63/564,223, filed March 12, 2024, entitled "INJECTION NEEDLE CAP REMOVAL TOOLS AND INJECTION TESTING SYSTEMS." The entirety of U.S. Provisional Patent Application Serial No. 63/564,223 is expressly incorporated herein by reference.

### FIELD OF THE DISCLOSURE

This disclosure relates generally to injection device testing, and more particularly, to injection needle cap removal tools and injector testing systems.

### BACKGROUND

Injection testing systems may test one or more aspects of injection devices, including autoinjectors, for aspects such as cap removal force, plunger actuation force, injection depth, injection time, needle retraction, and/or delivered dose.

### SUMMARY

Injection needle cap removal tools and injector testing systems are disclosed, substantially as illustrated by and described in connection with at least one of the figures, as set forth more completely in the claims.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG. 1 is an example injection testing system to perform testing of injection devices, in accordance with aspects of this disclosure.
FIG. 2 is a block diagram of an example injection testing system including an injection needle cap removal tool, in accordance with aspects of this disclosure.
FIG. 3A is a perspective view of an example implementation of the positioning plate and cap removal tool of FIG. 2.
FIG. 3B is an exploded view of the positioning plate and cap removal tool of FIG. 2.
FIGS. 4A and 4B are perspective views of the base of the example cap removal tool of FIGS. 3A and 3B.
FIGS. 5A and 5B are perspective views of the example cap removal tool of FIGS. 3A and 3B including an attached cap removal adapter.
FIG. 6 is a front elevation view of the example positioning plate and cap removal tool of FIGS. 3A and 3B, and further including an alignment tool inserted into the cap removal tool for positioning of the cap removal tool with respect to the positioning plate.
FIG. 7 is a perspective view of the example alignment tool of FIG. 6.
FIG. 8A is an exploded view of the example cap removal tool of FIGS. 3A and 3B including an attached injector alignment plate.
FIG. 8B is a perspective view of the example cap removal tool and injector alignment plate of FIG. 8A holding an example injector in position for actuation.
FIG. 9 is a flowchart representative of an example method to align the cap removal tool of FIGS. 3A and 3B and perform a cap removal force measurement.
FIG. 10 is a flowchart representative of an example method to align the cap removal tool of FIGS. 3A and 3B and perform an injection measurement using an injector alignment plate.
The figures are not necessarily to scale. Wherever appropriate, similar or identical reference numerals are used to refer to similar or identical components.

### DETAILED DESCRIPTION

Some injectors, such as autoinjectors, include a safety cap or similar cover to protect from accidental exposure to the needle of the injector in the event of an actuation of the injector. The force involved in removing the cap may be subject to certain requirements, such as upper and/or lower limits on the removal force. Conventional injector testing systems measure cap removal forces by gripping the cap using grips, and then moving the grips to separate the cap from the body of the injector. Some conventional injector testing systems used customized blocks that matched the contours of the entire cap and held the cap during the cap removal force measurement. However, the gripping forces applied by conventional injector testing systems cause distortion in the cap, thereby changing the force required to remove the cap. Disclosed example injector testing systems include a cap removal tool which improves the accuracy and repeatability of cap removal force measurements. In some examples, the cap removal tool includes a cap removal surface which retains the cap with reduced or eliminated distortion while the body of the injector is separated from the cap. Some disclosed examples further include alignment tools and procedures for correctly aligning the cap removal tool with a load string.

Disclosed example cap removal tools may be used with an autoinjector testing system, and include: a cap removal adapter having a cap removal aperture; and a base, including: first alignment features configured to align the cap removal tool with an actuator of the autoinjector testing system; and second alignment features configured to removably attach the cap removal adapter to the base and align the cap removal adapter with the actuator.

In some example cap removal tools, the base includes a seat and a flange, the cap removal adapter configured to abut the flange such that the cap removal adapter is spaced apart from the seat. In some example cap removal tools, the flange has a first width and the cap removal aperture has a second width less than the first width. Some example cap removal tools further include an alignment tool configured to mate with the base and to convey force from the actuator to the base. In some example cap removal tools, the base includes an alignment slot configured to receive a protrusion of the alignment tool.

Disclosed example injector testing systems include: a positioning plate having a first section configured to contact an injector; a cap removal tool coupled to a second section of the positioning plate, the cap removal tool comprising a cap removal surface facing the positioning plate; grips configured to grasp a body of the injector when the injector is positioned such that a cap of the injector abuts the cap removal surface; and an injector positioner configured to move the grips to separate the body of the injector from the cap.

In some example injector testing systems, the cap removal tool remains stationary while the injector positioner moves the grips to separate the body of the injector from the cap. Some example injector testing systems, further include a force sensor coupled to the injector positioner and configured to measure a load resulting from removal of the cap from the body. In some example injector testing systems, the force sensor is configured to measure an actuation force applied by an injector actuator to expel fluid from the injector.

In some example injector testing systems, the cap removal tool includes a base having first alignment features configured to couple the cap removal tool to the positioning plate. In some example injector testing systems, the cap removal surface is removable from the base. In some example injector testing systems, the first alignment features include slots configured to allow adjustment of the base with respect to the positioning plate. In some example injector testing systems, the base includes an alignment slot configured to receive a protrusion of an alignment tool, and the alignment tool is configured to extend from the alignment slot to the grips and to apply force from the grips to adjust the base with respect to the positioning plate.

Some example injector testing systems further include a positioning plate actuator configured to move the positioning plate from a first position in which the cap removal tool is aligned with the grips to a second position in which the first section is aligned with the grips. Some example injector testing systems further include an injector actuator configured to actuate the injector to expel contents of the injector via a needle of the injector while the injector is in contact with the first section of the positioning plate. In some example injector testing systems, the cap removal surface is removable and replaceable with an injector alignment tool configured to be coupled to the cap removal tool, and includes an injector alignment aperture configured to hold the injector in alignment with an injection aperture in the first section of the positioning plate.

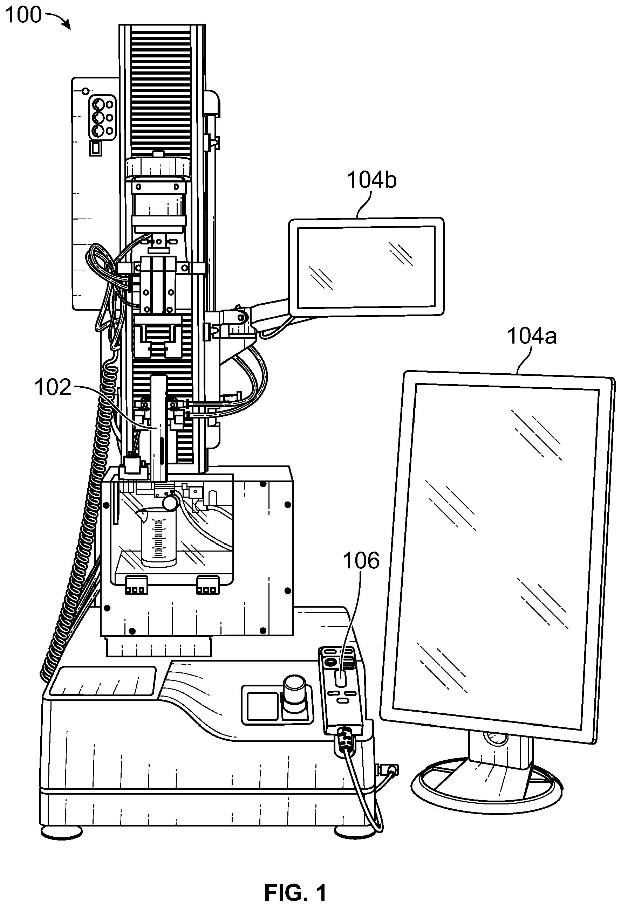

FIG. 1 is an example injection testing system 100 to perform testing on injection devices, such as an autoinjector 102. The example injection testing system 100 may be configured, for example, to perform some or all tests to evaluate requirements of the ISO 11608-5 standard. The example injection testing system 100 may be, for example, a universal testing system configured for injection testing.

The example injection testing system 100 of FIG. 1 includes positioning device(s) (e.g., to position the autoinjector 102 in one or more positions and/or orientations for automated testing), actuator(s) (e.g., to actuate components of the autoinjector 102, to actuate the positioning device(s), to position and/or orient test devices, etc.), and/or sensors to measure aspects of the autoinjector 102 during testing (e.g., load sensors to measure actuation force(s), auditory sensors to detect audible events), mass scales to measure an expelled dose, displacement and/or position sensors to trigger testing steps and/or measure displacement of components of the autoinjector 102, etc.). The example injection testing system 100 further includes one or more user interface devices, such as display 104 and input devices 106.

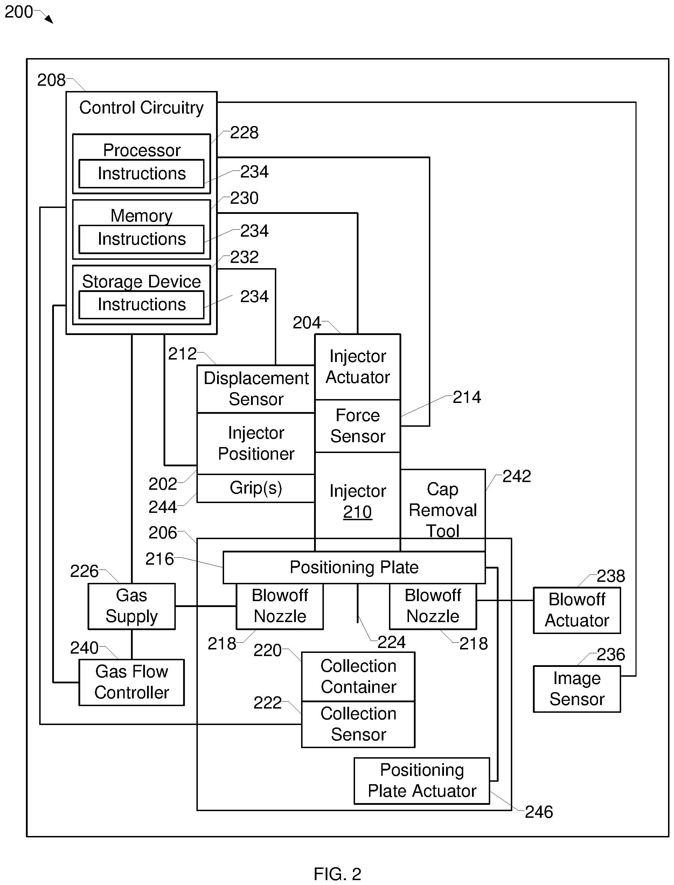

FIG. 2 is a block diagram of an example injection testing system 200 including a cap removal tool 242. The example injection testing system 200 may be used to implement some or all of the components of the injection testing system 100 of FIG. 1.

The example injection testing system 200 includes an injector positioner 202, an injector actuator 204, an injection collector 206, and control circuitry 208. The injector positioner 202 positions and/or orients an injector 210 (e.g., an autoinjector) for one or more tests in the injection testing system 200. For example, the injector positioner 202 may grasp the injector 210 and move and/or rotate the injector 210 for testing. The injector positioner 202 and/or the position of the injector 210 may be measured by one or more displacement sensor(s) 212, which provides displacement and/or position information to the control circuitry 208.

The injector actuator 204 actuates one or more aspects of the injector 210, such as a plunger or other injection mechanism of the injector 210. The force applied by the injector actuator 204 and/or the injector positioner 202 may by measured by a force sensor 214, which provides force measurements to the control circuitry 208. The force sensor 214 may measure, for example, forces involved in actuating the injector 210 (e.g., to expel fluid from the injector 210), forces involved in removing a cap from the injector 210, and/or any other resistance forces during actuations and/or strains on the injector 210. The force sensor 214 may be a load cell or other type of force sensor.

The injection collector 206 includes a loading surface (e.g., a positioning plate 216), blowoff nozzles 218, and a collection container 220. The collection container 220 and the injector 210 are positioned such that, when the injector 210 is actuated to expel fluid contained in the injector 210, the fluid is expelled into the collection container 220. A collection sensor 222 measures the mass and/or volume collected in the collection container 220, and provides a mass or volume measurement to the control circuitry 208.

The positioning plate 216 allows a needle 224 of the injector 210 to extend through the positioning plate 216 toward the collection container 220. The positioning plate 216 may block the body 225 of the injector 210 from extending through the positioning plate 216 using appropriately sized apertures for the needle 224 and body 225 of the injector 210. To test the dispensing of the contained fluid, the injector positioner 202 may position the injector 210 to contact or abut the positioning plate 216, such that the needle 224 extends through the aperture of the positioning plate 216. When the injector 210 is positioned, the injector 210 may be actuated (e.g., manually, or automatically via the injector actuator 204) to expel the contents of the injector 210 into the collection container 220.

While the examples disclosed herein use the positioning plate 216 as a loading surface, other examples may use different types of loading surfaces against which the injector 210 can be actuated to expose the needle 224 and/or expel the contents of the injector 210. For example, rods or other structural members which are positioned to contact a body of the injector 210 on a top side of the loading surface, and to avoid obstructing the needle 224 may be used. In some such examples, the blowoff nozzles 218 may be coupled to another surface, or otherwise adjustably supported, within the injection collector 206 adjacent the bottom side of the loading surface and/or adjacent the location the needle 224.

At the completion of actuation of the injector 210, the blowoff nozzles 218 are controlled to blow the last drop of fluid from at or near the tip of the needle into the collection container 220. A gas supply 226 provides a gas, such as nitrogen or air, to the blowoff nozzles 218. The gas supply 226 may be, for example, a compressed gas source, a pneumatic pump, or a blower. The blowoff nozzles 218 may be positioned and/or oriented to adjust a location at which the blowoff gas strikes the needle 224, and/or may be positioned and/or oriented to cause the blowoff gas to strike the needle 224 over a range of needle lengths.

Some injectors include a safety cap or similar cover to protect from accidental exposure to the needle 224 of the injector 210 in the event of an actuation of the injector 210. The force involved in removing the cap may be subject to certain requirements, such as upper and/or lower limits on the removal force. To aid in measuring the cap removal force, the example testing system 200 further includes a cap removal tool 242, which may be attached to the positioning plate 216. In contrast with prior cap removal methods such as gripping the cap with grips, the example cap removal tool 242 reduces or eliminates distortion forces on the cap, which can affect the measured removal force.

The grips 244 are coupled to the injector positioner 202 for positioning the injector 210. The grips 244 may be electric, pneumatic, and/or hydraulic grips, and are actuated by the injector positioner 202. The grips 244 may include one or more sets or pairs of grips. Example grips which may implement the grips 244 are disclosed in U.S. Provisional Patent Application No. 63/558,971, filed February 28, 2024, entitled "GRIPS FOR INJECTOR TESTING SYSTEMS," and U.S. Provisional Patent Application No. 63/588,550, filed October 6, 2023, entitled "GRIPS FOR INJECTOR TESTING SYSTEMS." The entireties of U.S. Provisional Patent Application No. 63/558,971 and U.S. Provisional Patent Application No. 63/588,550 are incorporated herein by reference.

In some examples, the testing system 200 includes a positioning plate actuator 246 to control a positioning of the positioning plate 216 with respect to a tool chain (e.g., the injector positioner 202, the grips 244, etc.). For example, the positioning plate actuator 246 may position different sections of the positioning plate 216 in alignment with the injector positioner 202 for different operations or portions of an injector test. For example, a first section of the positioning plate 216 may be used for cap removal (e.g., via the cap removal tool 242) and a second section of the positioning plate 216 may be used for actuation of the injector 210 and collection of fluid.

The injector positioner 202, the injector actuator 204, and the positioning plate actuator 246 may be implemented using any number of motors or actuators (e.g., electric motors, pneumatic or hydraulic actuators, etc.) and/or transmissions (e.g., to transmit force from one actuator to one or more types of movement).

The example control circuitry 208 may be a general-purpose computer, a laptop computer, a tablet computer, and/or any other type of processing system configured to communicate with the sensors and actuators of the injection testing system 200. For example, the control circuitry 208 includes a processor 228, memory 230, and a storage device 232. The example processor 228 may be any general purpose central processing unit (CPU) from any manufacturer. In some other examples, the processor 228 may include one or more specialized processing units, such as RISC processors with an ARM core, graphic processing units, digital signal processors, and/or system-on-chips (SoC). The processor 228 executes machine readable instructions 234 that may be stored locally at the processor (e.g., in an included cache or SoC), in the memory (e.g., a random access memory or other volatile memory, a read only memory or other non-volatile memory such as FLASH memory, and/or in the storage device 232. The example storage device 232 may be a hard drive, a solid state storage drive, a hybrid drive, a RAID array, and/or any other mass data storage device.

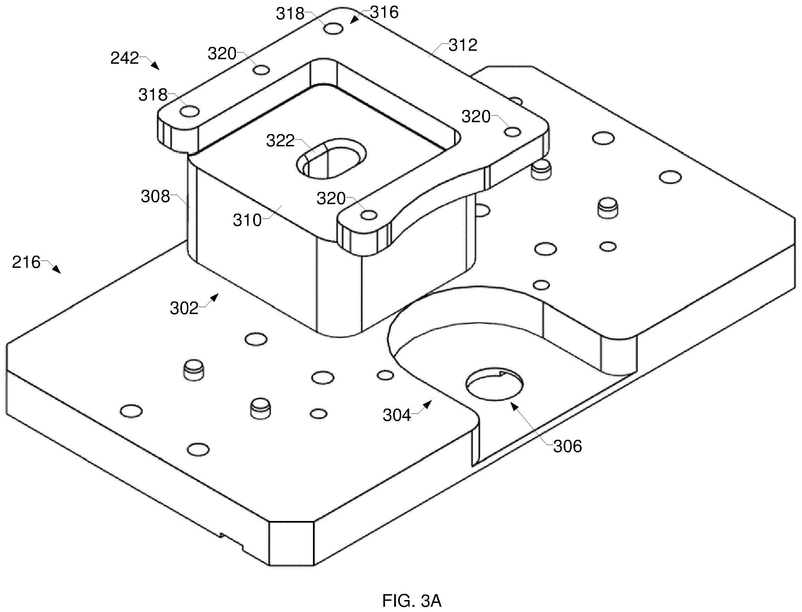

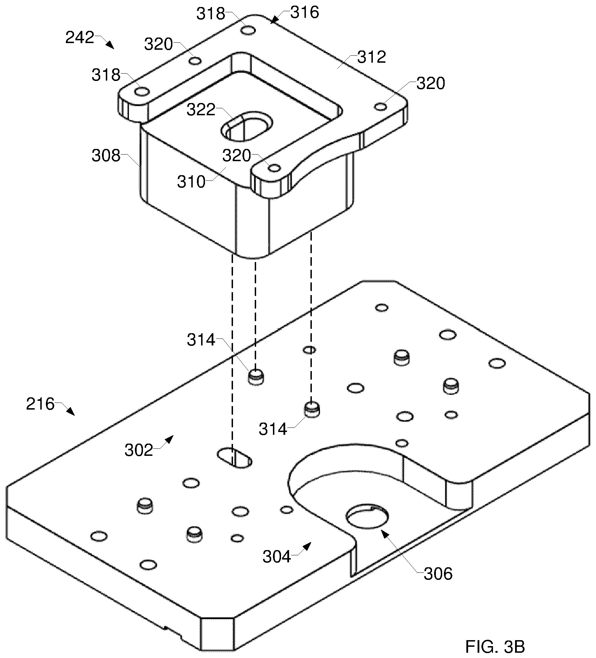

FIG. 3A is a perspective view of an example implementation of the positioning plate 216 and cap removal tool 242 of FIG. 2. FIG. 3B is an exploded view of the positioning plate 216 and cap removal tool 242 of FIG. 2. The example cap removal tool 242 is coupled to a first section 302 of the positioning plate 216, and a second section 304 of the positioning plate includes an injection aperture 306 for the needle 224 to extend through the positioning plate for an injector actuation.

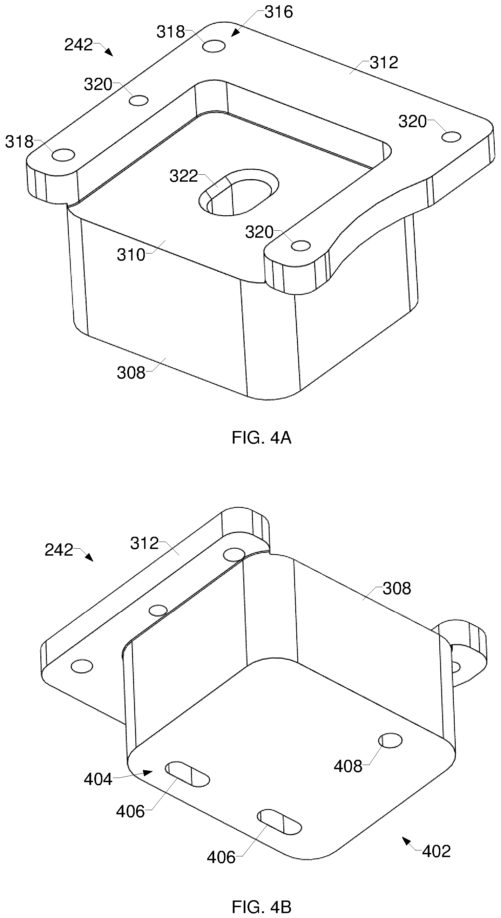

FIGS. 4A and 4B are perspective views of the base of the example cap removal tool of FIGS. 3A and 3B. The example cap removal tool 242 includes a base 308, which has a seat 310 and a flange 312 surrounding the seat 310 and raised up from the seat 310. The cap removal tool 242 may be machined from a single block of material (e.g., steel, plastic) and/or constructed using multiple components (e.g., separate pieces for the base 308 and the flange 312 welded or otherwise attached together).

As illustrated in FIG. 4B, a second end 402 of the base 308 includes first alignment features 404. The example first alignment features 404 include alignment slots 406 which allow for relative movement between the base 308 and protrusions 314 in the positioning plate 216 (FIG. 3B). The example first alignment features 404 further include a threaded hole 408, which may be used to secure the base 308 to the positioning plate 216 in a desired alignment via a bolt or other threaded fastener.

The flange 312 includes second alignment features 316 that may be used to attach a cap removal adapter. The example second alignment features 316 include alignment holes 318 (e.g., non-threaded holes) and attachment holes 320 (e.g., threaded holes for securing with a bolt). While example second alignment features 316 are shown, there may be more or fewer alignment holes 318 and attachment holes 320, and/or the attachment holes 320 may be non-threaded for use with a clamp, bolt and nut, and/or other clamping or attachment techniques.

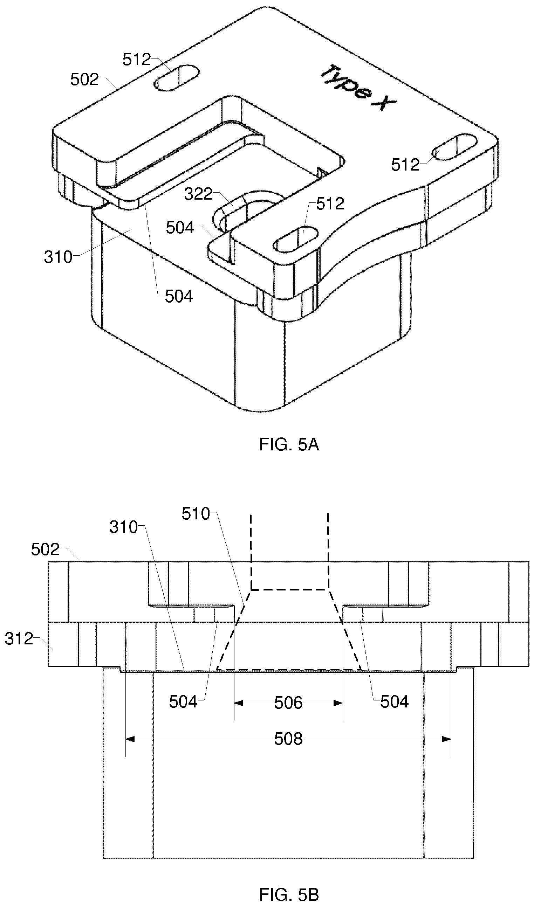

FIGS. 5A and 5B are perspective views of the example cap removal tool 242 of FIGS. 3A and 3B including an attached cap removal adapter 502. The cap removal adapter 502 is attached to the second alignment features 316 on the flange 312 while abutting the surface of the flange 312. The cap removal adapter 502 includes a cap removal surface 504 defined by an inner perimeter. The width 506 of the cap removal surface 504 is smaller than the width 508 of the seat 310. Due to the separation between the cap removal surface 504 and the seat 310, a portion of a cap 510 of an injector 210 may be inserted between the seat 310 and the cap removal surface 504.

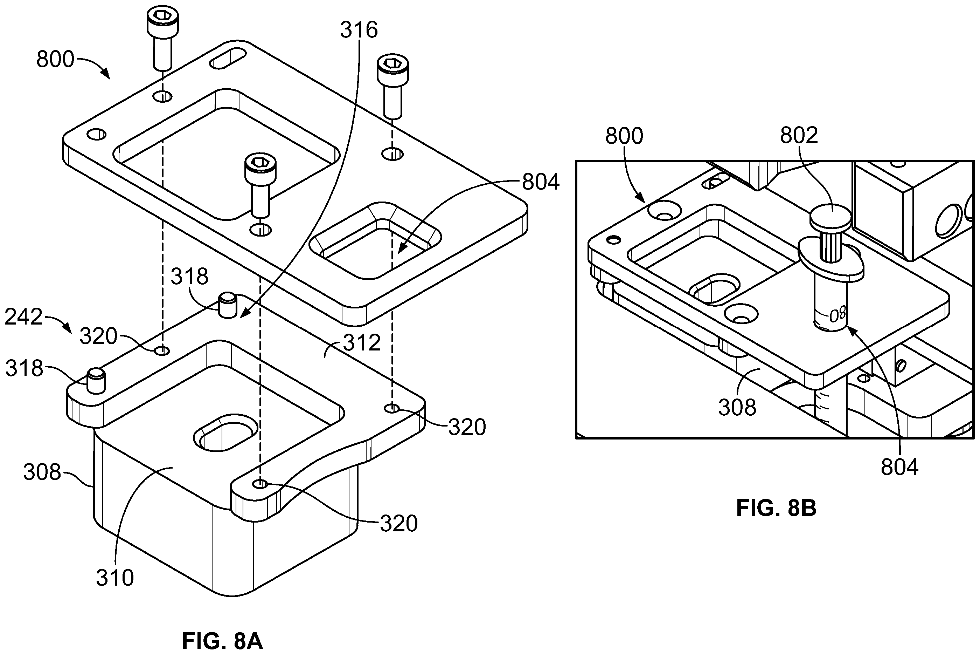

The cap removal adapter 502 is removable and/or replaceable to allow for use with different types of injectors and injector caps. For example, different cap removal adapters 502 may be attached that have different widths 506 and/or different separation between the cap removal surface 504 and the seat 310. Additionally or alternatively, other types of adapters may be attached to the base 308 to provide other functions, such as alignment and/or stabilization of injectors (as illustrated in FIGS. 8A and 8B).

The cap removal adapter 502 is coupled to the flange 312 via slots 512, which align with respective second alignment features 316 of the flange 312 (e.g., the threaded holes 320). For example, bolts may be inserted through the slots 512 and screwed into the flange 312 to secure the cap removal adapter 502 to the flange 312. The example slots 512 are elongated to allow for adjustment of the cap removal adapter 502 with respect to the flange 312 and the seat 310. The cap removal adapter 502 may include additional holes or slots to align with the alignment holes 318.

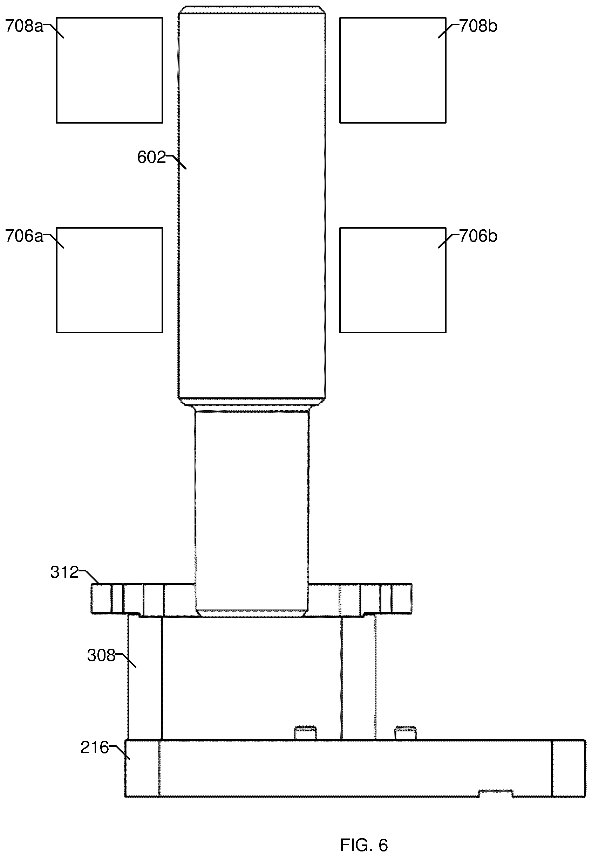

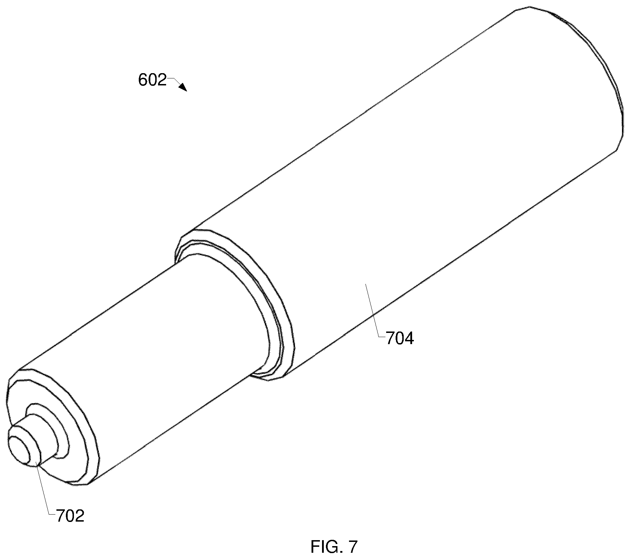

The example cap removal tool 242 further includes an alignment slot 322, which receives a corresponding protrusion of an alignment tool. FIG. 6 is a front elevation view of the example positioning plate 216 and cap removal tool 242 of FIGS. 3A and 3B, and further including an alignment tool 602 inserted into the cap removal tool 242 for positioning of the cap removal tool 242 with respect to the positioning plate 216. FIG. 7 is a perspective view of the example alignment tool 602 of FIG. 6. As illustrated in FIG. 7, the alignment tool 602 includes a protrusion 702, which fits into the alignment slot 322. The alignment tool 602 further includes a body 704, which extends from the seat 310 for engagement by example grips 706a, 706b, 708a, 708b. The grips 706a-708b may implement the grips 244 of FIG. 2.

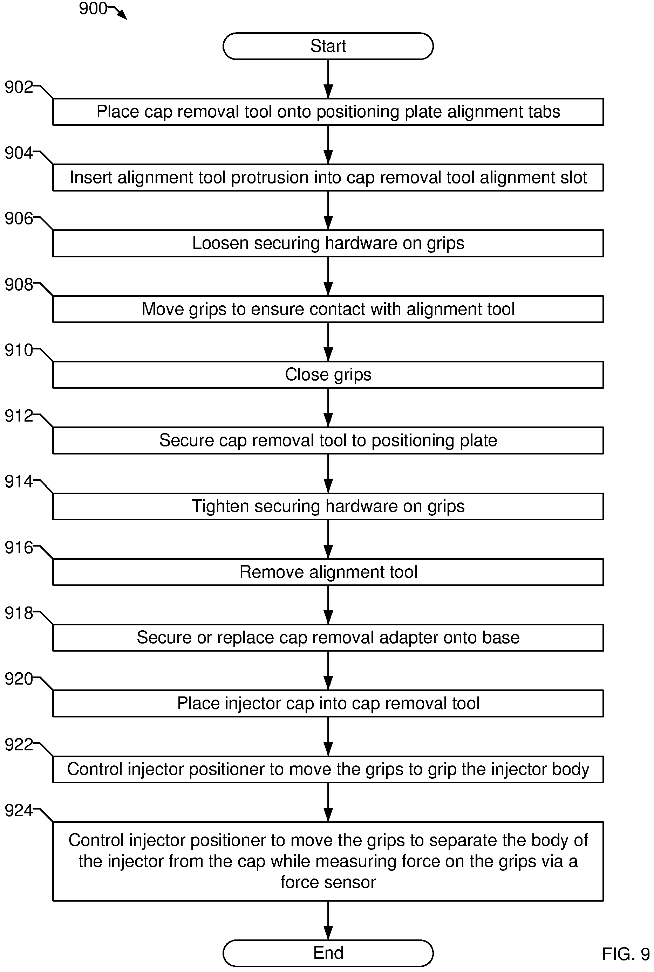

The cap removal tool 242 and the grips may be aligned with the grips 706a, 706b, 708a, 708b using the alignment tool 602 and an alignment process. The alignment slot 322 is elongated in a first direction and fits the protrusion 702 in a second direction with little tolerance to cause the alignment tool 602 to urge the cap removal tool 242 into alignment with the grips 706a, 706b, 708a, 708b during the alignment process. In the illustrated example, the alignment process involves: 1) loosening the connections between the cap removal tool 242 and the positioning plate 216 and loosening the attachments of the grips 706a, 706b, 708a, 708b; 2) closing the grips 706a, 706b, 708a, 708b onto the alignment tool 602 to cause the grips 706a, 706b, 708a, 708b and/or the cap removal tool 242 to shift into alignment; and 3) tightening the cap removal tool 242 to the positioning plate 216 and securing the attachments of the grips 706a, 706b, 708a, 708b. While loosened, the grips 706a-708b may be permitted to shift forward, backward, up, and/or down, as force is applied by the grips 706a-708b to the alignment tool 602, to compensate for any misalignment (e.g., due to tolerances). As a result of the shifting, the grips 706a-708b and the cap removal tool 242 are aligned with a same testing axis (e.g., a load string axis). A flowchart representative of an example method to perform the alignment is disclosed below with reference to FIG. 9.

Either or both pairs of grips 706a, 706b, 708a, 708b may be used to grasp the injector 210 during measurements, based on the type of injector 210 and/or the type of measurement or test being performed.

FIG. 8A is an exploded view of the example cap removal tool 242 of FIGS. 3A and 3B including an attached injector alignment plate 800. FIG. 8B is a perspective view of the example cap removal tool 242 and injector alignment plate 800 of FIG. 8A holding an example injector 802 in position for actuation. The example injector alignment plate 800 is attachable to the second alignment features 316 of the flange 312 in a similar manner as the cap removal adapter 502.

The injector alignment plate 800 includes an injector alignment aperture 804, which has a cross-section configured to support an injector 802 having a corresponding cross-section. For example, the injector alignment aperture 804 may hold the injector 802 upright and/or in alignment with the grips 244 while the needle of the injector 802 extends through the injection aperture 306. The injector alignment plate 800 may be replaced with different injector alignment plates 800 having different cross-sections for the injector alignment aperture 804.

FIG. 9 is a flowchart representative of an example method 900 to align the cap removal tool of FIGS. 3A and 3B and perform a cap removal force measurement.

At block 902, the cap removal tool 242 is placed onto alignment tabs (e.g., protrusions 314) of the positioning plate 216. At block 902, the cap removal tool 242 is not secured to the positioning plate 216, and is capable of relative movement with respect to the positioning plate 216 (e.g., the alignment slots 406 may slide over the protrusions 314).

At block 904, the alignment tool 602 (e.g., the protrusion 702) is inserted into the alignment slot 322 of the cap removal tool 242.

At block 906, securing hardware for the grips 706a-708b are loosened, which may allow for relative movement between each of the grips 706a-708b and mountings or actuators supporting the grips 706a-708b. By loosening, the grips 706a-708b may be permitted to shift in a same direction as the relative movement direction of the cap removal tool 242.

At block 908, the grips 706a-708b are actuated (e.g., by the injector positioner 202 of FIG. 2, which may be controlled manually and/or by the control circuitry 208) to ensure contact with the alignment tool 602. For example, the grips 706a-708b along a length of the alignment tool 602 to provide sufficient contact between the grips 706a-708b and the alignment tool 602 when the grips 706a-708b are closed.

At block 910, the grips 706a-708b are actuated (e.g., by the injector positioner 202 of FIG. 2, which may be controlled manually and/or by the control circuitry 208) to close on the alignment tool 602. Closing of the grips 706a-708b causes the grips 706a-708b and/or the cap removal tool 242 to shift as needed in along parallel directions to align the cap removal tool 242 with a load string of the testing system 200.

At block 912, the cap removal tool 242 is secured to the positioning plate 216 to prevent further relative movement. At block 914, the grips 706a-708b are secured to prevent further movement with respect to the supports and/or actuators. Following block 914, the cap removal tool 242 is aligned with the load string and the direction of actuation of the grips 706a-708b.

At block 916, the alignment tool is removed from the cap removal tool 242. At block 918, a cap removal adapter 502 is secured or replaced onto the base 308 (e.g., onto the flange 312 via securing hardware). The cap removal adapter 502 may be selected for a specific type of injector 210 and/or injector cap.

At block 920, an injector cap 510 is placed into the cap removal tool 242. The cap 510 may be seated on the seat 310 and at least partially below a cap removal surface 504 of the secured cap removal adapter 502.

At block 922, the control circuitry 208 controls the injector positioner 202 to move the grips 708a-708b to grip the body of the injector 210. In some cases, the lower grips 706a-706b are first actuated to help align the injector 210 (e.g., adjust tilting) prior to actuating the top grips 708a-708b for grasping of the injector 210. When the top grips 708a-708b are closed, the lower grips 706a-706b are then opened so that the top grips 708a-708b can be actuated for cap removal. At block 924, the control circuitry 208 controls the injector positioner 202 to move the grips 708a-708b to separate the body of the injector 210 from the cap 510. For example, the injector positioner 202 may actuate the grips away from the seat 310 (while the grips 708a-708b grasp the body of the injector 210). The body of the injector 210 moves with the grips, while the cap 510 is prevented from moving with the body by the cap removal surface 504. While the injector positioner 202 actuates the grips 708a-708b, the control circuitry 208 measures or monitors the force on the grips 708a-708b via the force sensor 214 to determine a removal force involved in removing the cap 510 from the body of the injector 210. The example method 900 then ends.

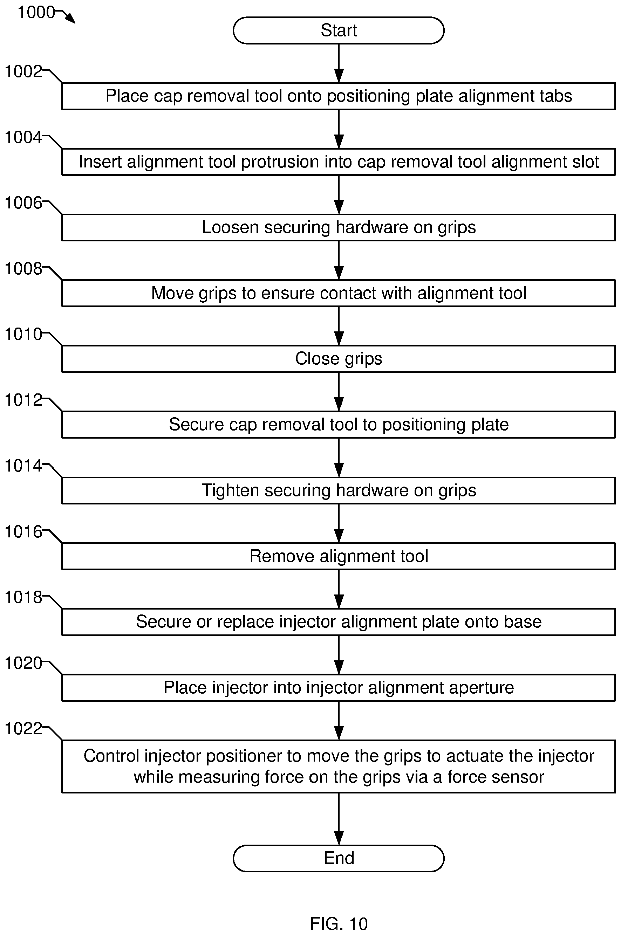

FIG. 10 is a flowchart representative of an example method 1000 to align the cap removal tool of FIGS. 3A and 3B and perform an injection measurement using an injector alignment plate (e.g., the injector alignment plate 800 of FIGS. 8A and 8B).

At block 1002, the cap removal tool 242 is placed onto alignment tabs (e.g., protrusions 314) of the positioning plate 216. At block 1002, the cap removal tool 242 is not secured to the positioning plate 216, and is capable of relative movement with respect to the positioning plate 216 (e.g., the alignment slots 406 may slide over the protrusions 314).

At block 1004, the alignment tool 602 (e.g., the protrusion 702) is inserted into the alignment slot 322 of the cap removal tool 242.

At block 1006, securing hardware for the grips 706a-708b are loosened, which may allow for relative movement between each of the grips 706a-708b and mountings or actuators supporting the grips 706a-708b. By loosening, the grips 706a-708b may be permitted to shift in a same direction as the relative movement direction of the cap removal tool 242.

At block 1008, the grips 706a-708b are actuated (e.g., by the injector positioner 202 of FIG. 2, which may be controlled manually and/or by the control circuitry 208) to ensure contact with the alignment tool 602. For example, the grips 706a-708b along a length of the alignment tool 602 to provide sufficient contact between the grips 706a-708b and the alignment tool 602 when the grips 706a-708b are closed.

At block 1010, the grips 706a-708b are actuated (e.g., by the injector positioner 202 of FIG. 2, which may be controlled manually and/or by the control circuitry 208) to close on the alignment tool 602. Closing of the grips 706a-708b causes the grips 706a-708b and/or the cap removal tool 242 to shift as needed in along parallel directions to align the cap removal tool 242 with a load string of the testing system 200.

At block 1012, the cap removal tool 242 is secured to the positioning plate 216 to prevent further relative movement. At block 1014, the grips 706a-708b are secured to prevent further movement with respect to the supports and/or actuators. Following block 1014, the cap removal tool 242 is aligned with the load string and the direction of actuation of the grips 706a-708b.

At block 1016, the alignment tool is removed from the cap removal tool 242. At block 1018, an injector alignment plate 800 is secured or replaced onto the base 308 (e.g., onto the flange 312 via securing hardware). The injector alignment plate 800 may be selected for a specific type of injector 802.

At block 1020, an injector 802 is placed into the injector alignment aperture 804 of the injector alignment plate 800. The injector 802 may be further placed into contact with the positioning plate 216, such that a needle 224 of the injector 802 extends through the injection aperture 306.

At block 1022, the control circuitry 208 controls the injector positioner 202 to move the grips 706a-708b to actuate the injector 802 positioned in the injector alignment plate 800. While the injector 802 is actuated, the example force sensor 214 may sense the force on the grips to actuate the injector 802.

In some examples, after placement of the injector 802 into the alignment aperture 804 (block 1020) and prior to the actuation of the injector 802 (block 1022), the grips 706a-708b may perform a cap removal from the injector 802. For example, after the injector is inserted into aperture 804, the top grips 708a-708b close onto the injector 802 and move the injector 802 a predetermined distance to place the cap of the injector 802 in a location to be grasped by the lower grips 706a-706b. The lower grips 706a-706b grip onto the cap of the injector 802, and the top grips 708a-708b then move up to perform the cap removal. In some examples, the positioning plate actuator 246 moves the positioning plate 216 to position the base 308 below the cap, and the lower grips 706a-706b dispose of the cap into the base by releasing the cap. The positioning plate actuator 246 may then move the positioning plate 216 to position the injection aperture 306 below the injector 802 to continue the injection measurement.

The example method 1000 then ends.

The present methods and systems may be realized in hardware, software, and/or a combination of hardware and software. The present methods and/or systems may be realized in a centralized fashion in at least one computing system, or in a distributed fashion where different elements are spread across several interconnected computing systems. Any kind of computing system or other apparatus adapted for carrying out the methods described herein is suited. A typical combination of hardware and software may include a general-purpose computing system with a program or other code that, when being loaded and executed, controls the computing system such that it carries out the methods described herein. Another typical implementation may comprise an application specific integrated circuit or chip. Some implementations may comprise a non-transitory machine-readable (e.g., computer readable) medium (e.g., FLASH drive, optical disk, magnetic storage disk, or the like) having stored thereon one or more lines of code executable by a machine, thereby causing the machine to perform processes as described herein. As used herein, the term "non-transitory machine-readable medium" is defined to include all types of machine readable storage media and to exclude propagating signals.

As utilized herein the terms "circuits" and "circuitry" refer to physical electronic components (i.e. hardware) and any software and/or firmware ("code") which may configure the hardware, be executed by the hardware, and or otherwise be associated with the hardware. As used herein, for example, a particular processor and memory may comprise a first "circuit" when executing a first one or more lines of code and may comprise a second "circuit" when executing a second one or more lines of code. As utilized herein, "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z". As utilized herein, the term "exemplary" means serving as a non-limiting example, instance, or illustration. As utilized herein, the terms "e.g.," and "for example" set off lists of one or more non-limiting examples, instances, or illustrations. As utilized herein, circuitry is "operable" to perform a function whenever the circuitry comprises the necessary hardware and code (if any is necessary) to perform the function, regardless of whether performance of the function is disabled or not enabled (e.g., by a user-configurable setting, factory trim, etc.).

While the present method and/or system has been described with reference to certain implementations, it will be understood by those skilled in the art that various changes may be made and equivalents may be substituted without departing from the scope of the present method and/or system. For example, block and/or components of disclosed examples may be combined, divided, re-arranged, and/or otherwise modified. In addition, many modifications may be made to adapt a particular situation or material to the teachings of the present disclosure without departing from its scope. Therefore, the present method and/or system are not limited to the particular implementations disclosed. Instead, the present method and/or system will include all implementations falling within the scope of the appended claims, both literally and under the doctrine of equivalents.

Certain embodiments of the invention are described in the following clauses:
Clause 1. A cap removal tool for an autoinjector testing system, the cap removal tool comprising:
   a cap removal adapter having a cap removal aperture; and
   a base, comprising:
      first alignment features configured to align the cap removal tool with an actuator of the autoinjector testing system; and
      second alignment features configured to removably attach the cap removal adapter to the base and align the cap removal adapter with the actuator.
Clause 2. The cap removal tool as defined in clause 1, wherein the base comprises a seat and a flange, the cap removal adapter configured to abut the flange such that the cap removal adapter is spaced apart from the seat.
Clause 3. The cap removal tool as defined in clause 2, wherein the flange has a first width and the cap removal aperture has a second width less than the first width.
Clause 4. The cap removal tool as defined in clause 1, further comprising an alignment tool configured to mate with the base and to convey force from the actuator to the base.
Clause 5. The cap removal tool as defined in clause 4, wherein the base comprises an alignment slot configured to receive a protrusion of the alignment tool.
Clause 6. An injector testing system, comprising:
   a positioning plate having a first section configured to contact an injector;
   a cap removal tool coupled to a second section of the positioning plate, the cap removal tool comprising a cap removal surface facing the positioning plate;
   grips configured to grasp a body of the injector when the injector is positioned such that a cap of the injector abuts the cap removal surface; and
   an injector positioner configured to move the grips to separate the body of the injector from the cap.
Clause 7. The injector testing system as defined in clause 6, wherein the cap removal tool remains stationary while the injector positioner moves the grips to separate the body of the injector from the cap.
Clause 8. The injector testing system as defined in clause 6, further comprising a force sensor coupled to the injector positioner and configured to measure a load resulting from removal of the cap from the body.
Clause 9. The injector testing system as defined in clause 8, wherein the force sensor is configured to measure an actuation force applied by an injector actuator to expel fluid from the injector.
Clause 10. The injector testing system as defined in clause 6, wherein the cap removal tool comprises a base having first alignment features configured to couple the cap removal tool to the positioning plate.
Clause 11. The injector testing system as defined in clause 10, wherein the cap removal surface is removable from the base.
Clause 12. The injector testing system as defined in clause 10, wherein the first alignment features comprise slots configured to allow adjustment of the base with respect to the positioning plate.
Clause 13. The injector testing system as defined in clause 12, wherein the base comprises an alignment slot configured to receive a protrusion of an alignment tool, the alignment tool configured to extend from the alignment slot to the grips and to apply force from the grips to adjust the base with respect to the positioning plate.
Clause 14. The injector testing system as defined in clause 6, further comprising a positioning plate actuator configured to move the positioning plate from a first position in which the cap removal tool is aligned with the grips to a second position in which the first section is aligned with the grips.
Clause 15. The injector testing system as defined in clause 14, further comprising an injector actuator configured to actuate the injector to expel contents of the injector via a needle of the injector while the injector is in contact with the first section of the positioning plate.
Clause 16. The injector testing system as defined in clause 6, wherein the cap removal surface is removable and replaceable with an injector alignment tool configured to be coupled to the cap removal tool and comprising an injector alignment aperture configured to hold the injector in alignment with an injection aperture in the first section of the positioning plate.

## Claims

1. A cap removal tool for an autoinjector testing system, the cap removal tool comprising:
a cap removal adapter having a cap removal aperture; and
a base, comprising:
first alignment features configured to align the cap removal tool with an actuator of the autoinjector testing system; and
second alignment features configured to removably attach the cap removal adapter to the base and align the cap removal adapter with the actuator.

2. The cap removal tool as defined in claim 1, wherein the base comprises a seat and a flange, the cap removal adapter configured to abut the flange such that the cap removal adapter is spaced apart from the seat.

3. The cap removal tool as defined in claim 2, wherein the flange has a first width and the cap removal aperture has a second width less than the first width.

4. The cap removal tool as defined in any one of claims 1 to 3, further comprising an alignment tool configured to mate with the base and to convey force from the actuator to the base.

5. The cap removal tool as defined in claim 4, wherein the base comprises an alignment slot configured to receive a protrusion of the alignment tool.

6. An injector testing system, comprising:
a positioning plate having a first section configured to contact an injector;
a cap removal tool coupled to a second section of the positioning plate, the cap removal tool comprising a cap removal surface facing the positioning plate;
grips configured to grasp a body of the injector when the injector is positioned such that a cap of the injector abuts the cap removal surface; and
an injector positioner configured to move the grips to separate the body of the injector from the cap.

7. The injector testing system as defined in claim 6, wherein the cap removal tool remains stationary while the injector positioner moves the grips to separate the body of the injector from the cap.

8. The injector testing system as defined in claim 6 or 7, further comprising a force sensor coupled to the injector positioner and configured to measure a load resulting from removal of the cap from the body, and optionally
wherein the force sensor is configured to measure an actuation force applied by an injector actuator to expel fluid from the injector.

9. The injector testing system as defined in any one of claims 6 to 8, wherein the cap removal tool comprises a base having first alignment features configured to couple the cap removal tool to the positioning plate.

10. The injector testing system as defined in claim 9, wherein the cap removal surface is removable from the base.

11. The injector testing system as defined in claim 9 or 10, wherein the first alignment features comprise slots configured to allow adjustment of the base with respect to the positioning plate.

12. The injector testing system as defined in claim 11, wherein the base comprises an alignment slot configured to receive a protrusion of an alignment tool, the alignment tool configured to extend from the alignment slot to the grips and to apply force from the grips to adjust the base with respect to the positioning plate.

13. The injector testing system as defined in any one of claims 6 to 12, further comprising a positioning plate actuator configured to move the positioning plate from a first position in which the cap removal tool is aligned with the grips to a second position in which the first section is aligned with the grips.

14. The injector testing system as defined in any one of claims 6 to 13, further comprising an injector actuator configured to actuate the injector to expel contents of the injector via a needle of the injector while the injector is in contact with the first section of the positioning plate.

15. The injector testing system as defined in any one of claims 6 to 14, wherein the cap removal surface is removable and replaceable with an injector alignment tool configured to be coupled to the cap removal tool and comprising an injector alignment aperture configured to hold the injector in alignment with an injection aperture in the first section of the positioning plate.
